# EUROPEAN PATENT APPLICATION

(11) **EP 4 706 605 A2**
(43) Date of publication of application: **11.03.2026**
(21) Application number: 25219659.7
(22) Date of filing: 21.03.2022
(51) Int. Cl.: A61F 5/453

(54) **FLUID COLLECTION DEVICES HAVING A MULTI-PART ADHESIVE COVER, AND RELATED SYSTEMS AND METHODS**

(30) Priority: 24.03.2021 US 202163165273 P
(62) Divisional of application: 22715846.6
(71) Applicant: Purewick Corporation, El Cajon, California 92020 (US)
(72) Inventor: NEWTON, Camille Rose, Bonsall, California, 92003 (US)
(74) Representative: Hoffmann Eitle

(57) **Abstract**

Examples relate to fluid collection devices having multiple adhesive covers, and related systems and methods. In some embodiments, a fluid collection device includes a fluid impermeable barrier defining an opening sized to receive a penis therethrough. The fluid collection device includes an attachment system secured to the outer surface of the fluid impermeable barrier. The attachment system includes one or more adhesive regions extending at least partially around the opening such that portions of the one or more adhesive regions are positioned on opposing sides of the opening. The attachment system also includes two adhesive covers removably secured to the one or more adhesive regions and positioned such that the opening is positioned between the two adhesives covers.

## Description

### CROSS-REFERENCE TO RELATED APPLICATIONS

This application claims priority to U.S. Provisional Patent Application No. 63/165,273 filed on March 24, 2021, the disclosure of which is incorporated herein, in its entirety, by this reference.

### BACKGROUND

An individual may have limited or impaired mobility such that typical urination processes are challenging or impossible. For example, the individual may have surgery or a disability that impairs mobility. In another example, the individual may have restricted travel conditions such as those experienced by pilots, drivers, and workers in hazardous areas. Additionally, fluid collection from the individual may be needed for monitoring purposes or clinical testing.

Bed pans and urinary catheters, such as a Foley catheter, may be used to address some of these circumstances. However, bed pans and urinary catheters have several problems associated therewith. For example, bed pans may be prone to discomfort, spills, and other hygiene issues. Urinary catheters may be uncomfortable, painful, and may cause urinary tract infections.

Thus, users and manufacturers of fluid collection devices continue to seek new and improved devices, systems, and methods to collect urine.

### SUMMARY

Embodiments disclosed herein are related to fluid collection devices having a multi-part adhesive cover, and related systems and methods. In an embodiment, a fluid collection device includes a fluid impermeable barrier having a first portion, a second portion, and a chamber defined between the first portion and the second portion. The first portion of the fluid impermeable barrier includes an outer surface and an inner surface and defines an opening sized to receive a penis therethrough. The fluid collection device also includes a fluid permeable body positioned at least partially within the chamber. The fluid collection device also includes an attachment system secured to the outer surface of the fluid impermeable barrier. The attachment system includes one or more adhesive regions extending at least partially around the opening such that portions of the one or more adhesive regions are positioned on at least opposing sides of the opening. The one or more adhesive regions are configured to adhere to skin of a user. The attachment system also includes two adhesive covers removably secured to the one or more adhesive regions and positioned such that the opening is positioned between the two adhesives covers.

In an embodiment, a method of collecting fluid such as urine includes positioning a penis of a user at least partially in a chamber defined by a fluid collection device and through an opening defined by a first portion of a fluid impermeable barrier of the fluid collection device. The fluid impermeable barrier includes a second portion and a chamber defined between the first portion and the second portion of the fluid impermeable barrier. The fluid collection device includes a fluid permeable body positioned at least partially within the chamber. The method includes removing a first adhesive cover from a first adhesive region secured to an outer surface of the first portion of the fluid impermeable barrier. The first adhesive region extends at least partially around the opening such that the first adhesive region is positioned at least partially between the opening and a first side portion of the fluid impermeable barrier. The method includes adhering the first adhesive region to at least one of pelvic, suprapubic, or abdominal skin of the user on a first side of the penis. The method includes, after adhering the first adhesive region to the at least one of pelvic, suprapubic, or abdominal skin of the user on the first side of the penis, removing a second adhesive cover from a second adhesive region secured to the outer surface of the first portion of the impermeable barrier. The second adhesive region extends at least partially around the opening such that the second adhesive region is positioned at least partially between the opening and a second side portion of the fluid impermeable barrier opposite to the first side portion. The method includes adhering the second adhesive region to at least one of pelvic, suprapubic, or abdominal skin of the user on a second side of the penis.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG. 1** is a block diagram of a fluid collection system, according to an embodiment.
**FIG. 2A** is a front view of a fluid collection device, according to an embodiment.
**FIG. 2B** is a rear view of the fluid collection device of **FIG. 2A****.**
**FIG. 2C** is a cross-sectional view of the fluid collection device of **FIG. 2A** along line 2C-2C.
**FIG. 3A** is a rear view of a portion of a fluid collection device, according to an embodiment.
**FIG. 3B** is a rear view of a portion of a fluid collection device, according to an embodiment.
**FIG. 3C** is a rear view of a portion of a fluid collection device, according to an embodiment.
**FIG. 3D** is a rear view of a portion of a fluid collection device, according to an embodiment.
**FIG. 3E** is a rear view of a portion of a fluid collection device, according to an embodiment.
**FIGS. 4A** and **4B** are isometric side views of a fluid collection device being secured to a user, according to an embodiment.
**FIG. 5** is a flow diagram of a method for collecting fluid, according to an embodiment.

### DETAILED DESCRIPTION

Embodiments disclosed herein are related to fluid collection devices having a multi-part adhesive cover, and related systems and methods of use. Conventional fluid collection devices may include an adhesive and a single adhesive cover or backing removably secured to the adhesive. The adhesive may be configured to secure the fluid collection device to the user, such as securing the adhesive to skin on at least one of a pelvic, a suprapubic, or an abdominal region around the penis of the user. The single adhesive cover of conventional fluid collection devices may extend around opposing portions of the opening into the fluid collection device. Thus, to secure the fluid collection device to the user, the entire single adhesive cover of a conventional fluid collection device may need to be removed before the fluid collection device is positioned on the user. Placement and securement of the adhesive with the entire adhesive cover removed may be difficult and uncomfortable for both the user and the caregiver. For example, a non-erect penis tends to lay to the side and to get the penis through the opening of the fluid collection device, a hand of the user or caregiver is required to hold the top of the penis. This holding of the top of the penis may become difficult when the hand holding the penis is not able to go through the opening of the fluid collection device with the penis. When the hand lets go of the penis, the penis may flop to the side and come in contact with the uncovered adhesive. The hand assisting in securing the fluid collection device to the user also may come in contact with the uncovered adhesive. The uncovered adhesive, then, may adhere or stick to the hand and/or the penis rather than (or in addition to) the skin around the penis, without the penis actually going through the opening of the fluid collection device as intended.

At least one, some, or all embodiments of fluid collection devices described herein may include one or more adhesive regions and two (or more) adhesive covers removably secured to the one or more adhesive regions. This configuration of one or more adhesive regions and two or more adhesive covers results in the technical effect of securing the one or more adhesive regions to the skin of the user one at a time, and reducing the chance that the one or more adhesive regions secure to undesired locations, such as a hand or the penis itself. For example, a penis may be inserted through the opening in the fluid collection device before any of the two or more adhesive covers are removed from the one or more adhesive regions. Because the one or more adhesive regions remain covered while the penis is inserted through the opening, the one or more adhesive regions will not adhere to the penis or the gloves as the penis is inserted through the opening of the fluid collection device. Once the penis is inserted through the opening, the user or caregiver may remove a first adhesive from a first portion of the one or more adhesive regions and secure that first portion of the one or more adhesive regions to the skin of the user before removing the second adhesive cover. Once the first portion of the one or more adhesive regions is secured to the user, the user or caregiver may then remove the second adhesive cover from a second portion of the one or more adhesive regions and then secure that second portion of the one or more adhesive regions to the skin of the user. The first and second portions of the one or more adhesive regions may be at least partially positioned on opposite sides of the opening in the fluid collection device effective to secure the fluid collection device to the user with the penis extending through the opening.

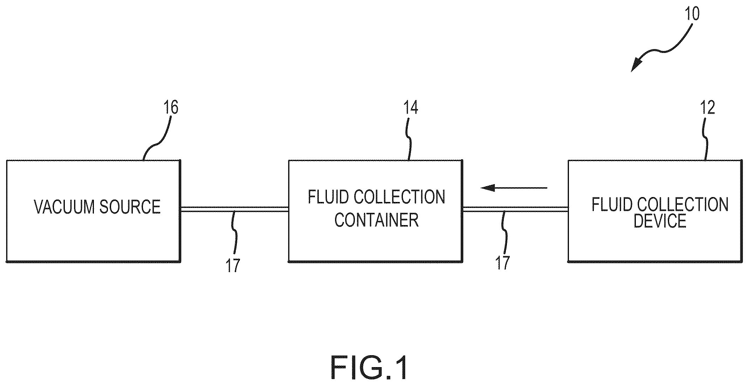

**FIG. 1** is a block diagram of a fluid collection system 10, according to an embodiment. The fluid collection system 10 may be included in any of the embodiments of fluid collection systems described herein. The system 10 includes a fluid (*e.g*., urine) collection device 12 (*e.g.,* any of the fluid collection assemblies disclosed herein), a fluid collection container 14, and a vacuum source 16 (*e.g.,* pump). The fluid collection device 12, the fluid collection container 14, and the vacuum source 16 may be fluidly coupled to each other via one or more tubes 17 or conduits. For example, fluid collection device 12 may be operably coupled to one or more of the fluid collection container 14 or the vacuum source 16 via the tube 17. In some embodiments, the vacuum source 16 may be secured directly to the fluid collection container 14. Fluid (*e.g*., urine or other bodily fluids) collected in the fluid collection device 12 may be removed from the fluid collection device 12 via the tube 17 secured to the fluid collection device 12. Suction force may be introduced into the chamber of the fluid collection device 12 via the inlet of the tube 17 responsive to suction (*e.g*., vacuum) force applied at the outlet of the tube 17.

The suction force may be applied to the outlet of the tube 17 by the vacuum source 16 either directly or indirectly. The suction force may be applied indirectly via the fluid collection container 14. For example, the outlet of the tube 17 may be disposed within or fluidly coupled to an interior region of the fluid collection container 14 and an additional tube 17 may extend from the fluid collection container 14 to the vacuum source 16. Accordingly, the vacuum source 16 may apply suction to the fluid collection device 12 via the fluid collection container 14. The suction force may be applied directly via the vacuum source 16. For example, the outlet of the tube 17 may be disposed within the vacuum source 16. An additional tube 17 may extend from the vacuum source 16 to a point outside of the fluid collection device 12, such as to the fluid collection container 14. In such examples, the vacuum source 16 may be disposed between the fluid collection device 12 and the fluid collection container 14.

The fluid collection container 14 is sized and shaped to retain a fluid therein. The fluid collection container 14 may include a bag (*e.g*., drainage bag), a bottle or cup (*e.g*., collection jar), or any other enclosed container for storing bodily fluid(s) such as urine. In some examples, the tube 17 may extend from the fluid collection device 12 and attach to the fluid collection container 14 at a first point therein. An additional tube 17 may attach to the fluid collection container 14 at a second point thereon and may extend and attach to the vacuum source 16. Accordingly, a vacuum (*e.g*., suction) may be drawn through fluid collection device 12 via the fluid collection container 14. Fluid, such as urine, may be drained from the fluid collection device 12 using the vacuum source 16.

The vacuum source 16 may include one or more of a manual vacuum pump, and electric vacuum pump, a diaphragm pump, a centrifugal pump, a displacement pump, a magnetically driven pump, a peristaltic pump, or any source configured to produce a vacuum (*e.g*., negative pressure). The vacuum source 16 may provide a vacuum or suction to remove fluid from the fluid collection device 12. In some examples, the vacuum source 16 may be powered by one or more of a power cord (*e.g*., connected to a power socket), one or more batteries, or even manual power (*e.g.,* a hand operated vacuum pump). In some examples, the vacuum source 16 may be sized and shaped to fit outside of, on, or within the fluid collection device 12. For example, the vacuum source 16 may include one or more miniaturized pumps or one or more micro pumps. The vacuum sources disclosed herein may include one or more of a switch, a button, a plug, a remote, or any other device suitable to activate the vacuum source 16. Aspects of the fluid collection system 10 may be included in any of the fluid collection systems described herein. For example, the fluid collection device 12 may include any of the fluid or urine collection devices described herein.

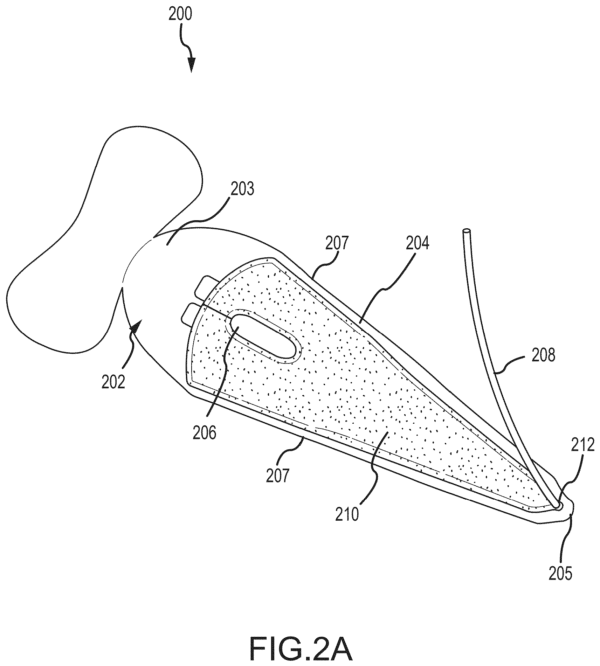

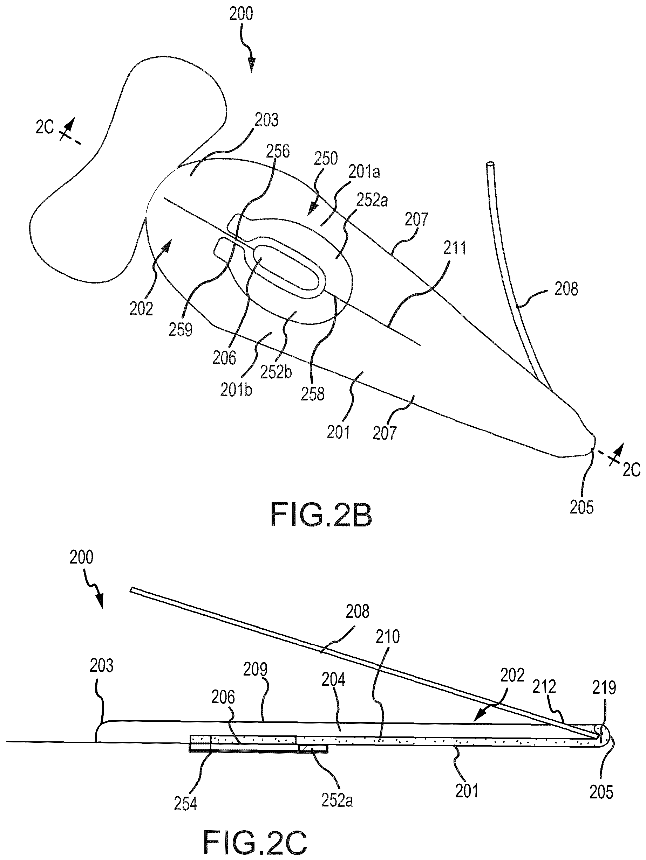

**FIG. 2A** is a front view of a male urine collection device 200 and **FIG. 2B** is a rear view of the male urine collection device 200, according to an embodiment. The urine collection device 200 described below is provided as an example of a urine collection device having an attachment system 250. The attachment systems described herein, however, may be secured or otherwise included in other urine collection devices configured differently than the urine collection device 200. For example, the attachment systems described herein may be similarly secured around the opening for a penis of other urine collection devices. The urine collection device 200 includes a fluid impermeable barrier 202 at least partially defining an opening 206 and a chamber 204 within the fluid collection device in fluid communication with the opening 206. In some embodiments, a bottom portion 201 of the fluid impermeable barrier 202 may define the opening 206 and an opposite or a top portion 209 (shown in **FIG. 2C**) of the fluid impermeable barrier 202 may define an aperture 212. In some embodiments, the bottom portion 201 of the fluid impermeable barrier 202 may include a slit 211 dividing a first side 201a of the bottom portion 201 and a second side 201b of the bottom portion 201. The slit 211 may extend from the opening 206 at least partially to the distal end region 205 of the fluid impermeable barrier. The slit 211 may extend from the opening 206 at least partially (e.g., entirely) to the proximal end region 203. However, in other embodiment, the slit 211 may be omitted. The fluid impermeable barrier 202 includes a proximal end region 203 and a distal end region 205. The opening 206 may be positioned proximate or closer to the proximal end region 203 than the distal end region 205 of the fluid impermeable barrier 202. In some embodiments, the fluid impermeable barrier 202 narrows between the proximal end region 203 and the distal end region 205. For example, the fluid impermeable barrier 202 (and the chamber 204) may include a substantially triangular front profile, with the distal end region 205 being at the narrow end or tip of the triangular profile. The fluid impermeable barrier 202 may include opposing side portions 207 (*e.g.,* side edges) extending at least partially between the distal end region 205 and the proximal end region 203. The fluid impermeable barrier 202 may include a substantially flexible fluid impermeable material, such as a fluid impermeable polymer (*e.g*., silicone, polypropylene, polyethylene, polyethylene terephthalate, a polycarbonate, etc.), polyurethane films, thermoplastic elastomer, oil, another suitable material, or combinations thereof. In some embodiments, the fluid impermeable barrier includes a paper-like fluid impermeable material or a fluid impermeable fabric.

The urine collection device 200 also includes a fluid permeable body 210 positioned within the chamber 204 and extending at least partially between the distal end region 205 and the proximal end region 203. The fluid permeable body 210 may be shaped generally complementary to the shape of the chamber of the fluid impermeable barrier 202. In some embodiments, the fluid permeable body 210 is spaced from the edges of the fluid impermeable barrier 202. In some embodiments, the fluid permeable body 210 is positioned to abut the edges of the fluid impermeable barrier 202 such that fluid impermeable barrier 202 retains fluid in the fluid permeable body 210 from the opening 206 to the distal end region 205.

The fluid permeable body 210 can be configured to wick and/or allow transport of fluid away from the opening 206, thereby preventing the fluid from escaping the chamber 204. The fluid permeable body 210 also can wick and/or allow transport of the fluid generally towards a sump 219 (shown in **FIG. 2C**) at the distal end region 205. The fluid permeable body 210 can include any material that can wick and/or allow transport of the fluid. The permeable properties referred to herein can be wicking, capillary action, diffusion, or other similar properties or processes, and are referred to herein as "permeable" and/or "wicking." Such "wicking" or other physical properties may exclude absorption into the fluid permeable body 210, such as not include adsorption of the bodily fluids into the fluid permeable body 210. Put another way, substantially no absorption or solubility of the bodily fluids into the material may take place after the material is exposed to the bodily fluids and removed from the bodily fluids for a time. While no absorption or solubility is desired, the term "substantially no absorption" may allow for nominal amounts of absorption and/or solubility of the bodily fluids into the fluid permeable body 210 (*e.g.,* absorbency), such as less than about 30 wt% of the dry weight of the fluid permeable body 210, less than about 20 wt%, less than about 10 wt%, less than about 7 wt%, less than about 5 wt%, less than about 3 wt%, less than about 2 wt%, less than about 1 wt%, or less than about 0.5 wt% of the dry weight of the fluid permeable body 210. In an embodiment, the fluid permeable body 210 may include at least one absorbent or adsorbent material.

The fluid permeable body 210 can include a one-way fluid movement fabric. As such, the fluid permeable body 210 can remove fluid from the area around the penis, thereby leaving the area and urethra dry. The fluid permeable body 210 can enable the fluid to flow generally towards the sump 219 and the tube 208 within the chamber 204. The fluid permeable body 210 can include a porous or fibrous material, such as hydrophilic polyolefin. In some embodiments, the fluid permeable body 210 consists of or consists essentially of a porous or fibrous material, such as hydrophilic polyolefin. Examples of polyolefin that can be used in the fluid permeable body 210 include, but are not limited to, polyethylene, polypropylene, polyisobutylene, ethylene propylene rubber, ethylene propylene diene monomer, or combinations thereof. Moreover, the fluid permeable body 210 can be manufactured according to various manufacturing methods, such as molding, extrusion, or sintering. The fluid permeable body 210 can include varying densities or dimensions.

In some embodiments, the fluid permeable body 210 can include two or more layers of fluid permeable materials. For example, the fluid permeable body 210 can include a fluid permeable membrane covering or wrapped around a fluid permeable support, with both the fluid permeable membrane and the fluid permeable support being disposed in the chamber 204. The fluid permeable membrane can cover or extend across at least a portion (*e.g.,* all) of at least the side of the fluid permeable support facing the penis of the user. The fluid permeable membrane and the fluid permeable support can be configured to wick any fluid away from the opening 206, thereby preventing the fluid from escaping the chamber 204 and promoting removal of the fluid through the tube 208.

The fluid permeable membrane and the fluid permeable support also can wick and/or allow transport of the fluid generally towards an interior of the chamber 204, such as the sump 219. The fluid permeable membrane can include any material that can wick the fluid. For example, the fluid permeable membrane can include fabric, such as a gauze (e*.g.,* a silk, linen, polymer based materials such as polyester, or cotton gauze), nylon (such as a spun nylon fibers), another soft fabric (*e.g*., jersey knit fabric or the like), or another smooth fabric (*e.g*., rayon, satin, or the like). Forming the fluid permeable membrane from gauze, soft fabric, and/or smooth fabric can reduce chaffing caused by the urine collection device 200. Other embodiments of fluid permeable membranes and fluid permeable supports are disclosed in U.S. Patent Application No. 15/612,325 filed on June 2, 2017; U.S. Patent Application No. 15/260,103 filed on September 8, 2016; U.S. Patent Application No. 15/611,587 filed on June 1, 2017; PCT Patent Application No. PCT/US19/29608, filed on April 29, 2019, the disclosure of each of which is incorporated herein, in its entirety, by this reference. In many embodiments, the fluid permeable body 210 includes a fluid permeable support including a porous spun nylon fiber structure and a fluid permeable wicking membrane including gauze at least partially enclosing the spun nylon fiber structure. For example, the fluid permeable body 210 may include a gauze or other wicking fabric positioned to contact the skin of the user. In some embodiments, the gauze or other wicking fabric is wrapped around a body of spun nylon fibers material and/or covering both sides of a substantially planar spun nylon fibers material. In some embodiments, the gauze or other wicking fabric covers the side of substantially planar spun nylon fibers material that is oriented towards the skin of the user.

The urine collection device 200 also includes a tube 208 extending into the chamber 204 and having an end positioned proximate to the distal end region 205 of the fluid impermeable barrier 202. A sump may be defined as the region or area between the tube opening and the distal end region 205 of the fluid impermeable barrier 202. Fluid discharged on the fluid permeable body 210 may flow to and pool in the sump for removal when a vacuum is applied on the tube 208. The tube 208 may extend through an aperture 212 in a top portion of the fluid impermeable barrier 202 and into the chamber 204. The aperture 212 may be positioned proximate to the distal end region 205 of fluid impermeable barrier 202. In some embodiments, the end of the tube 208 and/or the tube opening are positioned between the distal end region 205 and the aperture 212. In some embodiments, the aperture 212 is absent, and the tube 208 may exit the chamber 204 through the opening 206. U.S. Provisional Patent 63/067,542 describes embodiments of male external catheter urine collection devices, aspects of which may be used in any of the embodiments disclosed herein, the disclosure of which are incorporated in their entirety by this reference.

**FIG. 2B** is a rear view of a urine collection device 200, according to an embodiment. The urine collection device 200 may include an attachment system 250 secured to the bottom portion 201 of the fluid impermeable barrier 202. The attachment system 250 includes one or more adhesive regions 254 (shown in **FIG. 2C**) extending at least partially around the opening 206 such that portions of the one or more adhesive regions 254 are positioned on opposing sides of the opening 206. The one or more adhesive regions 254 may include any material and/or adhesive(s) configured to removably adhere to skin of the user. In some embodiments, the one or more adhesive regions 254 may include at least an acrylic or cyanoacrylate adhesive, a silicone adhesive, a hydrocolloid adhesive, a synthetic based rubber adhesive, or combinations thereof.

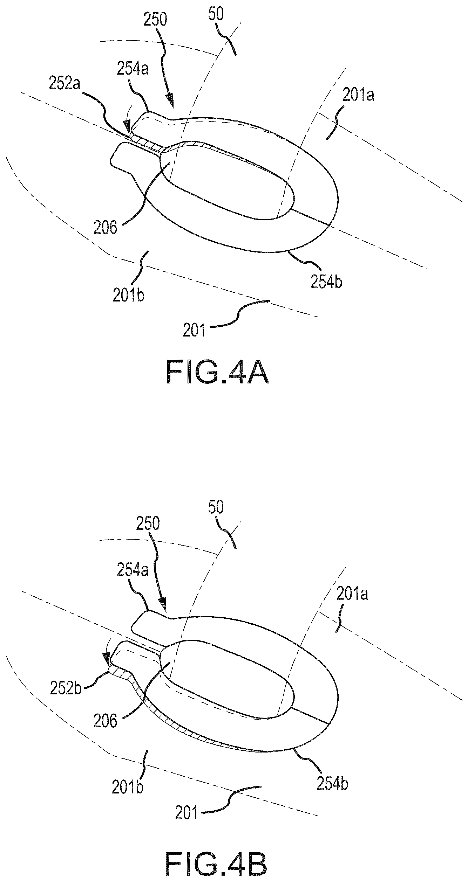

The attachment system 250 also includes two or more adhesive covers 252a, 252b removably secured to the one or more adhesive regions 254 and positioned such that the opening 206 is positioned between the two or more adhesives covers 252a, 252b. The two or more adhesive covers 252a, 252b may together cover substantially all of the one or more adhesive regions 254 such that the one or more adhesive regions 254 do not adhere to skin, gloves, etc., while the one or more covers 252a, 252b are removably secured to the one or more adhesive regions 254. Positioning of the two or more adhesives covers 252a, 252b on opposing sides or portions of the opening 206 allows a penis to be inserted into the opening 206 with the two or more adhesives covers 252a, 252b secured to the one or more adhesive regions 254. Once the penis has been inserted into the opening 206, the two or more adhesives covers 252a, 252b may be individually removed from the one or more adhesive regions 254 and secured to the skin of the user surrounding the penis one at a time, as described in greater detail in relation to **FIG. 4A-4B****,** below. In some embodiments, the two or more adhesive covers 252a, 252b may include three, four, five, six, or more adhesive covers removably secured to one or more adhesive regions 254. The one or more adhesive regions 254 may include two, three, four, five, six or more adhesive regions 254. In some embodiments, the urine collection device 250 may include the same number of adhesive covers 252 as adhesive regions 254 such that each adhesive cover is removably secured to a different and distinct adhesive region.

**FIG. 2C** is a cross-sectional view of the urine collection device 200 taken along line 2C-2C of **FIG. 2B****.** The two or more adhesives covers 252a, 252b may be removably secured to the one or more adhesive regions 254, and the one or more adhesive regions 254 may be secured to the outer surface of the bottom portion 201 of the fluid impermeable barrier 202. The fluid permeable body may be positioned on the inner surface of the bottom portion 201 of the fluid impermeable barrier 202. The bottom portion 201 and the top portion 209 of the fluid impermeable barrier 202 may define the chamber 204 in the urine collection device 200 for collecting urine discharged by the penis.

In some embodiments, the attachment system 250 is substantially horseshoeshaped. Returning to **FIG. 2B****,** the one or more adhesive regions 254 may be positioned between the opening 206 and each of the two side portions 207 (or side edges) and each of the adhesive covers 252a, 252b may be at least partially positioned on the one or more adhesive regions 254 between the opening 206 and a different side portion of the two side portions 207. In some embodiments, the one or more adhesive regions 254 and at least some of each of the two adhesive covers 252a, 252b are positioned at least partially between the opening 206 and the distal end region 205 of the fluid impermeable barrier 202. The two or more adhesive covers 252a, 252b may each include a terminating edge 258 positioned between the opening 206 and the distal end region 205. The terminating edge 258 of the two or more adhesive covers 252a, 252b may abut one another between the opening 206 and the distal end region 205. The attachment system 250 may include a gap or space 256 between terminating edges 259 or ends of the one or more adhesive regions 254 and the two or more adhesive covers 252a, 252b. The space 256 between terminating edges 259 of the one or more adhesive regions 254 and the two or more adhesive covers 252a, 252b may be positioned between the opening 206 and the proximal end region 203 of the fluid impermeable barrier 202. Thus, the space 256 may be generally opposite of the terminating edges 258 that abut one another, with the opening 206 positioned therebetween.

The one or more adhesive regions 254 may include a single continuous adhesive material including one or more (*e.g*., two) adhesive regions, areas, or portions. In some embodiments, the one or more adhesive regions 254 include two separate adhesive regions spaced from one another, each of the two adhesive covers 252a, 252b removably secured to a different one of the two adhesive regions.

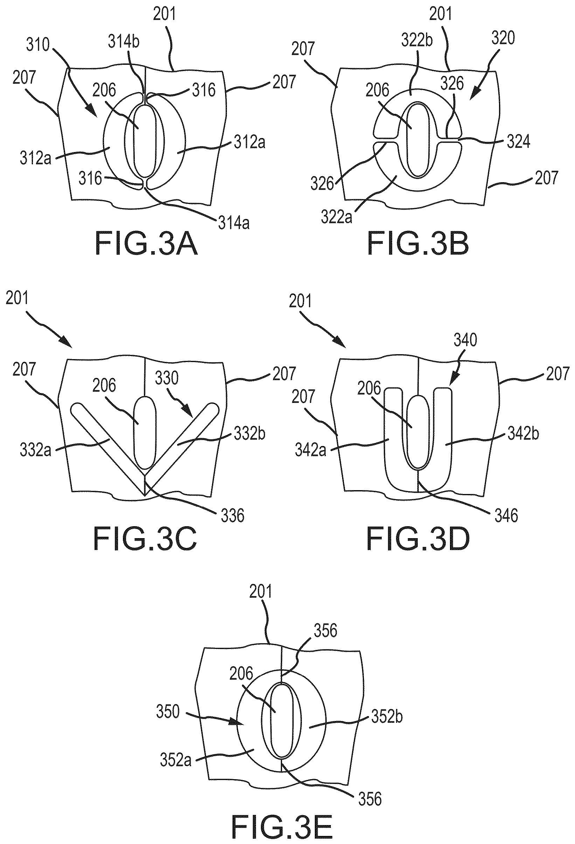

Other attachment systems may include other configurations and shapes. **FIGS. 3A-3E** are examples of attachment systems that may be used with urine collection devices, such as the urine collection device 200. Unless otherwise noted, the attachment systems of **FIGS. 3A-3E** may include any aspect of the attachment system 250, such as the one or more adhesive regions and two or more adhesive covers. The one or more adhesive regions may be secured to an outer surface of the bottom portion 201 of the fluid impermeable barrier 202.

Turning specifically to **FIG. 3A****,** an attachment system 310 may include two adhesive covers 312a, 312b removably secured to two adhesive regions (not visible underneath the two adhesive covers 312a, 312b. The two adhesive covers 312a, 312b and the two adhesive regions may be substantially arc-shaped complementary to one another. The two adhesive covers 312a, 312b (and the two adhesive regions) may each include two terminating edges 316 or ends. A gap 314 or space may be positioned between the terminating edges 316 of the two adhesive covers 312a, 312b (and the two adhesive regions). In some embodiments, the attachment system 310 is oriented such that a first space 314a is positioned between terminating edges 316 and also is positioned between the opening 206 and the distal end region 205 of the fluid impermeable barrier 202. In some embodiment, the attachment system 310 is oriented such at that a second space 314a is positioned between the opening 206 and the proximal end region 203 of the fluid impermeable barrier 202. In other embodiments, the terminating edges 316 may abut one another between the distal end region 205 and the opening 206 and/or between the proximal end region 203 and the opening 206.

**FIG. 3B** shows an attachment system 320 having two adhesive covers 322a, 322b removably secured to two adhesive regions (not visible underneath the two adhesive covers 322a, 322b), according to an embodiment. The attachment system 320 may be similar to the attachment system 310, but with terminating edges 326 and spaces 324 positioned between the opening 206 and the side portions 207 or edges of the fluid impermeable body 202.

**FIG. 3C** shows an attachment system 330 having two adhesive covers 332a, 332b secured to one or more adhesive regions (not visible underneath the two adhesive covers 332a, 332b), according to an embodiment. The two or more adhesive covers 332a, 332b and the one or more adhesive regions may be shaped generally complementary to one another. In some embodiments, the two adhesive covers 332a, 332b may together form a V-shape. The one or more adhesive regions may include a single, continuous adhesive region forming a V-shape or two adhesive regions forming a V-shape generally complementary to the V-shape formed by the two or more adhesive covers 332a, 332b. The two adhesive covers 332a, 332b may each include a terminating edge 336 that abuts the terminating edge 336 of the other adhesive cover proximate to the point of the V-shape. In some embodiments, the terminating edges 346 may abut one another between the distal end region 205 of the fluid impermeable barrier 202 and the opening 206. In some embodiments, the terminating edges 336 may abut one another between the proximal end region 203 and the opening 206. In some embodiments, the attachment system 330 is configured such that a space positioned between terminating edges 336 is positioned between the opening 206 and the distal end region 205 of the fluid impermeable barrier 202.

**FIG. 3D** shows an attachment system 340 having two adhesive covers 342a, 342b secured to one or more adhesive regions (not visible underneath the two adhesive covers 342a, 342b), according to an embodiment. The two or more adhesive covers 342a, 342b and the one or more adhesive regions may be shaped generally complementary to one another. In some embodiments, the two adhesive covers 342a, 342b may together form a U-shape. The one or more adhesive regions may include a single, continuous adhesive region forming a U-shape or two adhesive regions forming a U-shape generally complementary to the U-shape formed by the two or more adhesive covers 342a, 342b. The two adhesive covers 342a, 342b may each include a terminating edge 346 that abuts the terminating edge 346 of the other adhesive cover proximate to the base of the U-shape. In some embodiments, the terminating edges 346 may abut one another between the distal end region 205 of the fluid impermeable barrier 202 and the opening 206. In some embodiments, the terminating edges 346 may abut one another between the proximal end region 203 and the opening 206. In some embodiments, the attachment system 330 is configured such that a space positioned between terminating edges 346 is positioned between the opening 206 and the distal end region 205 of the fluid impermeable barrier 202.

**FIG. 3E** shows an attachment system 350 having two adhesive covers 352a, 352b secured to one or more adhesive regions (not visible underneath the two adhesive covers 352a, 352b), according to an embodiment. The two or more adhesive covers 352a, 352b and the one or more adhesive regions may be shaped generally complementary to one another. In some embodiments, the two adhesive covers 352a, 352b may together form an O-shape. The one or more adhesive regions may include a single, continuous adhesive region forming an O-shape or two adhesive regions forming an O-shape generally complementary to the O-shape formed by the two or more adhesive covers 352a, 352b. The two adhesive covers 352a, 352b may each include two terminating edges 356 that abut the two terminating edges 356 of the other adhesive cover. In some embodiments, terminating edges 356 may abut one another between the distal end region 205 of the fluid impermeable barrier 202 and the opening 206 and also may abut one another between the proximal end region 203 of the fluid impermeable barrier 202 and the opening 206.

**FIGS. 4A** and **4B** show the attachment system 250 being secured to a user, according to an embodiment. **FIGS. 4A** and 4B are shown with the fluid permeable body 210 removed for an unobstructed view of the attachment system 250 from inside the chamber 204 or above the fluid collection device 200. Although the attachment system 250 is shown in **FIGS. 4A** and **4B****,** other attachment systems 310, 320, 330, 340, 350 may be secured to a user similar or the same as shown in **FIGS. 4A** and **4B****.** The penis 50 of the user may be positioned at least partially in the chamber 204 and through the opening 206 in the bottom portion 201 of the fluid impermeable barrier 201 before either of the adhesive covers 252a, 252b are removed from the adhesive region 254. The slit 211 may provide a larger opening for positioning a flaccid penis 50 at least partially in the chamber 204 before securing the fluid collection device 200 to the user. Neither of the adhesive covers 252a, 252b are removed from the adhesive region 254 when inserting the penis into the opening 206, resulting in the technical effect of the adhesive region 254 not adhering to undesired locations during insertion of the penis 50, such as the hand or glove of the user or caregiver, clothes, or the penis 50 itself. To secure the fluid collection device 200 to the user, the second side 201b of the bottom portion 201 of the fluid impermeable barrier 202 may be pulled to the side towards the edge 207 to at least partially position the penis 50 in the chamber 204. Once the penis 50 is at least partially positioned in the chamber 204 through the opening 206 and the slit 211, at least part of a first side of the bottom portion 201 of the fluid impermeable barrier 202 may be lifted and a first adhesive cover 252a may be peeled or removed from a first portion 254a of the adhesive region 254, as shown in **FIG. 4A****.** The first portion 254a of the adhesive region 254 may then be adhered to the skin of the user near the penis 50, such as at least one of pelvic, suprapubic, or abdominal skin near the penis 50, before the second adhesive cover 252b is peeled from the adhesive region 254. With the first portion of the adhesive region 254 adhering to the skin of the user (under the first adhesive region 254a in **FIG. 4B**), at least part of a second side of the bottom portion 201 of the fluid impermeable barrier 202 may be lifted and the second adhesive cover 252b may be peeled or removed from a second portion of the adhesive region 254, as shown in **FIG. 4B****.** The second portion 254b of the adhesive region 254 may then be adhered to the skin of the user near the penis 50, such as at least one of pelvic, suprapubic, or abdominal skin near the user. With the first portion 254a and the second portion 254b of the adhesive region 254 secured to the user, the penis 50 may extend through the opening 206 into the chamber 204.

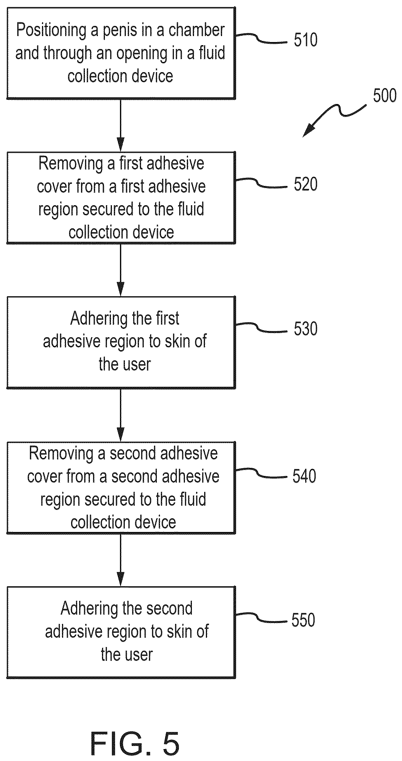

**FIG. 5** is a flow diagram of a method 500 for securing a fluid collection device on a user, according to an embodiment. The method 500 includes an act 510 of positioning a penis at least partially in a chamber and through an opening in a fluid collection device. The method also includes an act 520 of removing a first adhesive cover from a first adhesive region secured to the fluid collection device. The method also includes an act 530 of adhering the first adhesive region to skin of the user. The method also includes an act 540 of removing a second adhesive cover from a second adhesive region secured to the fluid collection device. The method also include an act 550 of adhering the second adhesive region to skin of the user. In some embodiments, the method 500 may be configured as a method of securing a fluid collection device to a user and also collecting fluid from the user, and the method may include an act of drawing fluid discharged in the chamber from the fluid collection device through a tube.

The act 510 may include positioning a penis of a user at least partially in a chamber defined by a fluid collection device and through an opening defined by a first portion of a fluid impermeable barrier of the fluid collection device. The fluid collection device may include any fluid collection device described herein, such as the urine collection device 200. The fluid impermeable barrier may include a second portion and a chamber defined between the first portion and the second portion of the fluid impermeable barrier and the fluid collection device may include a fluid permeable body positioned at least partially within the chamber.

The act 520 may include removing a first adhesive cover from a first adhesive region secured to an outer surface of the first portion of the fluid impermeable barrier. The first adhesive cover and the first adhesive region may be part of any of the attachment systems described herein, such as the attachment system 250, 310, 320, 330, 340, 350. The first adhesive region may extend at least partially around the opening such that the first adhesive region is positioned at least partially between the opening and a first side portion of the fluid impermeable barrier. In some embodiments, the act 520 of removing a first adhesive cover from a first adhesive region may include removing the first adhesive cover from the first adhesive region secured to the outer surface of the first portion of the fluid impermeable barrier between the opening and the first side portion of the fluid impermeable barrier and also between the opening and a distal end region of the fluid impermeable barrier.

The act 530 may include adhering the first adhesive region to skin of the user around or proximate to the base of the penis. For example, the act 530 may include adhering the first adhesive region to a first region of at least one of pelvic, suprapubic, or abdominal skin to the side of the penis.

The act 540 may include, after adhering the first adhesive region to at least one of pelvic, suprapubic, or abdominal skin of the user on the first side of the penis, removing a second adhesive cover from a second adhesive region secured to the outer surface of the first portion of the impermeable barrier. The second adhesive region may extend at least partially around the opening such that the second adhesive region is positioned at least partially between the opening and a second side portion of the fluid impermeable barrier opposite to the first side portion. In some embodiments, the act 540 of removing a second adhesive cover from a second adhesive region may include removing the second adhesive cover from the second adhesive region secured to the outer surface of the first portion of the fluid impermeable barrier between the opening and the second side portion of the fluid impermeable barrier and also between the opening and the distal end region of the fluid impermeable barrier.

The act 550 may include adhering the second adhesive region to skin of the user around or proximate to the base of the penis. For example, the act 550 may include adhering the second adhesive region to a second region of at least one of pelvic, suprapubic, or abdominal skin to the side of the penis such that the penis is positioned between the first region of pelvic skin and the second region of the at least one of pelvic, suprapubic, or abdominal skin.

The acts of the method 500 described above are for illustrative purposes. For example, the acts of the method 500 can be performed in different orders, split into multiple acts, modified, supplemented, or combined. In an embodiment, one or more of the act of the method 500 can be omitted from the method 500. Any of the acts of the method 500 can include using any of the portable urine collection systems disclosed herein.

As used herein, the term "about" or "substantially" refers to an allowable variance of the term modified by "about" or "substantially" by ±10% or ±5%. Further, the terms "less than," "or less," "greater than," "more than," or "or more" include, as an endpoint, the value that is modified by the terms "less than," "or less," "greater than," "more than," or "or more."

While various aspects and embodiments have been disclosed herein, other aspects and embodiments are contemplated. The various aspects and embodiments disclosed herein are for purposes of illustration and are not intended to be limiting. The following clauses define aspects of the disclosure which may or may not presently be clauseed.
1. A fluid collection device, comprising:
   a fluid impermeable barrier having a first portion, a second portion, and a chamber defined between the first portion and the second portion, the first portion including an outer surface and an inner surface and defining an opening sized to receive a penis therethrough;
   a fluid permeable body positioned at least partially within the chamber; and
   an attachment system secured to the outer surface of the fluid impermeable barrier, the attachment system including:
      one or more adhesive regions extending at least partially around the opening such that portions of the one or more adhesive regions are positioned on at least opposing sides of the opening, the one or more adhesive regions being configured to adhere to skin of a user; and
      two adhesive covers removably secured to the one or more adhesive regions and positioned such that the opening is positioned between the two adhesives covers.
2. The fluid collection device of clause 1, wherein the one or more adhesive regions include two adhesive regions spaced from one another, each of the two adhesive covers removably secured to a different one of the two adhesive regions.
3. The fluid collection device of any of clauses 1-2, wherein each of the two adhesive covers are substantially arc shaped.
4. The fluid collection device of clause 1, wherein:
   the fluid impermeable barrier includes a proximal end region, a distal end region, and two side regions extending at least partially between the proximal end region and the distal end region; and
   the opening of the fluid impermeable barrier is positioned between the distal end region and the proximal region and between the two side regions.
5. The fluid collection device of clause 4, wherein the one or more adhesive regions are positioned between the opening and each of the two side portions, and each of the adhesive covers is at least partially positioned on the one or more adhesive regions between the opening and a different side portion of the two side portions.
6. The fluid collection device of clause 5, wherein the one or more adhesive regions and at least a portion of each of the two adhesive covers are positioned at least partially between the opening and the distal end region.
7. The fluid collection device of clause 6, wherein the two adhesive covers each include a terminating edge positioned between the opening and the distal end region.
8. The fluid collection device of clause 7, wherein the terminating edge of the two adhesive covers abut one another between the opening and the distal end region.
9. The fluid collection device of any of clauses 5-8, wherein the one or more adhesive regions include a continuous adhesive region.
10. The fluid collection device of clause 9, wherein the continuous adhesive region is a substantially U-shaped continuous adhesive region and the two adhesive covers are positioned adjacent one another to form a U-shape substantially complementary to the U-shaped continuous adhesive region.
11. The fluid collection device of clause 9, wherein the continuous adhesive region is a substantially V-shaped continuous adhesive region and the two adhesive covers are positioned adjacent to one another to form a V-shape substantially complementary to the V-shaped continuous adhesive region.
12. The fluid collection device of clause 9, wherein the continuous adhesive region is a substantially O-shaped continuous adhesive region around the opening and the two adhesive covers are positioned adjacent to one another to form an O-shape substantially complementary to the O-shaped continuous adhesive region.
13. The fluid collection device of clause 4, wherein the one or more adhesive regions include two adhesive regions spaced from one another between the opening and the distal end region, each of the two adhesive covers removably secured to a different one of the two adhesive regions.
14. The fluid collection device of clause 13, wherein the two adhesive covers each include a terminating edge spaced from one another between the opening and the two adhesive regions.
15. The fluid collection device of any of clauses 13 or 14, wherein:
   at least a portion of the one or more adhesive regions is positioned between the opening and the proximal end region;
   a first adhesive cover of the two adhesive covers is removably secured to at least the portion of the one or more adhesive regions and positioned between the opening and the proximal end region;
   at least a portion of the one or more adhesive regions is positioned between the opening and the distal end region; and
   a second adhesive cover of the two adhesive covers is removably secured to at least the portion of the one or more adhesive regions and positioned between the opening and the distal end region.
16. The fluid collection device of any of clauses 4-8 or 13-15, wherein each of the two adhesive covers are substantially arc shaped.
17. The fluid collection device of any of clauses 1-16, further comprising a tube configured to provide fluid communication between the chamber and a vacuum source.
18. The fluid collection device of any of clauses 1-17, wherein the fluid permeable body includes a fluid permeable porous fiber structure and a wicking material positioned to be proximate skin of the user and between the skin of the user and the fluid permeable porous fiber structure.
19. A method of collecting fluid, the method comprising:
   positioning a penis of a user at least partially in a chamber defined by a fluid collection device and through an opening defined by a first portion of a fluid impermeable barrier of the fluid collection device, the fluid impermeable barrier having a second portion and a chamber defined between the first portion and the second portion of the fluid impermeable barrier and the fluid collection device including a fluid permeable body positioned at least partially within the chamber;
   removing a first adhesive cover from a first adhesive region secured to an outer surface of the first portion of the fluid impermeable barrier, the first adhesive region extending at least partially around the opening such that the first adhesive region is positioned at least partially between the opening and a first side portion of the fluid impermeable barrier;
   adhering the first adhesive region to at least one of pelvic, suprapubic, or abdominal skin of the user on a first side of the penis;
   after adhering the first adhesive region to the at least one of pelvic, suprapubic, or abdominal skin of the user on the first side of the penis, removing a second adhesive cover from a second adhesive region secured to the outer surface of the first portion of the impermeable barrier, the second adhesive region extending at least partially around the opening such that the second adhesive region is positioned at least partially between the opening and a second side portion of the fluid impermeable barrier opposite to the first side portion; and
   adhering the second adhesive region to at least one of pelvic, suprapubic, or abdominal skin of the user on a second side of the penis.
20. The method of clause 19, further comprising drawing fluid discharged in the chamber from the fluid collection device through a tube.
21. The method of any of clauses 19 or 20, wherein:
   removing a first adhesive cover from a first adhesive region secured to an outer surface of the first portion of the fluid impermeable barrier includes removing the first adhesive cover from the first adhesive region secured to the outer surface of the first portion of the fluid impermeable barrier between the opening and the first side portion of the fluid impermeable barrier and also between the opening and a distal end region of the fluid impermeable barrier; and
   removing a second adhesive cover from a second adhesive region secured to the outer surface of the first portion of the impermeable barrier includes removing the second adhesive cover from the second adhesive region secured to the outer surface of the first portion of the fluid impermeable barrier between the opening and the second side portion of the fluid impermeable barrier and also between the opening and the distal end region of the fluid impermeable barrier.

## Claims

1. A fluid collection device (200), comprising:
a fluid impermeable barrier (202) at least partially defining a chamber (204) and an opening (206) sized to receive a penis therethrough;
a planar fluid permeable body (210) positioned at least partially within the chamber; and
an attachment system (250, 310, 320, 330, 340, 350) secured to an outer surface of the fluid impermeable barrier, the attachment system including:
one or more adhesive regions (254) extending at least partially around the opening such that portions of the one or more adhesive regions are positioned on at least opposing sides of the opening, the one or more adhesive regions being configured to adhere to skin of a user; and
two adhesive covers (252a, 252b, 312a, 312b, 322a, 322b, 332a, 332b, 342a, 342b, 352a, 352b) removably secured to the one or more adhesive regions and positioned such that the opening is positioned between the two adhesive covers.

2. The fluid collection device of claim 1, wherein the one or more adhesive regions include two adhesive regions spaced from one another, each of the two adhesive covers (312a, 312b) removably secured to a different one of the two adhesive regions.

3. The fluid collection device of any of claims 1-2, wherein each of the two adhesive covers (312a, 312b) are substantially arc shaped.

4. The fluid collection device of claim 1, wherein:
the fluid impermeable barrier includes a proximal end region (203), a distal end region (205), and two side regions (207) extending at least partially between the proximal end region and the distal end region; and
the opening (206) of the fluid impermeable barrier is positioned between the distal end region (205) and the proximal region (203) and between the two side regions (207).

5. The fluid collection device of claim 4, wherein the one or more adhesive regions (254) are at least partially positioned between the opening (206) and each of the two side portions (207), and each of the adhesive covers (252a, 252b, 312a, 312b, 322a, 322b, 332a, 332b, 342a, 342b, 352a, 352b) is at least partially positioned on the one or more adhesive regions between the opening and a different side portion of the two side portions.

6. The fluid collection device of claim 5, wherein the one or more adhesive regions (254) and at least a portion of each of the two adhesive covers are positioned at least partially between the opening (206) and the distal end region (205).

7. The fluid collection device of claim 1, wherein the one or more adhesive regions (254) include a continuous adhesive region.

8. The fluid collection device of claim 7, wherein the continuous adhesive region is a substantially U-shaped continuous adhesive region and the two adhesive covers (342a, 342b) are positioned adjacent one another to form a U-shape substantially complementary to the U-shaped continuous adhesive region.

9. The fluid collection device of claim 7, wherein the continuous adhesive region is a substantially V-shaped continuous adhesive region and the two adhesive covers (332a, 332b) are positioned adjacent to one another to form a V-shape substantially complementary to the V-shaped continuous adhesive region.

10. The fluid collection device of claim 7, wherein the continuous adhesive region is a substantially O-shaped continuous adhesive region around the opening and the two adhesive covers (352a, 352b) are positioned adjacent to one another to form an O-shape substantially complementary to the O-shaped continuous adhesive region.

11. The fluid collection device of claim 4, wherein the one or more adhesive regions include two adhesive regions spaced from one another between the opening and the distal end region, each of the two adhesive covers removably secured to a different one of the two adhesive regions (254).

12. The fluid collection device of claim 11, wherein:
at least a portion of the one or more adhesive regions is positioned between the opening (206) and the proximal end region (203);
a first adhesive cover of the two adhesive covers is removably secured to at least the portion of the one or more adhesive regions and positioned between the opening and the proximal end region;
at least a portion of the one or more adhesive regions (254) is positioned between the opening and the distal end region; and
a second adhesive cover of the two adhesive covers is removably secured to at least the portion of the one or more adhesive regions and positioned between the opening and the distal end region.

13. The fluid collection device of any of claims 1-12, further comprising a tube (17) configured to provide fluid communication between the chamber (14) and a vacuum source (16).

14. A method of collecting fluid, the method comprising:
positioning a penis of a user at least partially in a chamber (204) at least partially defined by a fluid impermeable barrier of a fluid collection device (200) and through an opening (206) at least partially defined by the fluid impermeable barrier of the fluid collection device, the fluid collection device including a planar fluid permeable body (210) positioned at least partially within the chamber;
removing a first adhesive cover (252a, 312a, 322a, 332a, 342a, 352a) from a first adhesive region (254) secured to an outer surface of the fluid impermeable barrier, the first adhesive region extending at least partially around the opening such that the first adhesive region is positioned at least partially between the opening and a first side portion of the fluid impermeable barrier;
adhering the first adhesive region to at least one of pelvic, suprapubic, or abdominal skin of the user on a first side of the penis;
after adhering the first adhesive region to the at least one of pelvic, suprapubic, or abdominal skin of the user on the first side of the penis, removing a second adhesive cover (252b, 312b, 322b, 332b, 342b, 352b) from a second adhesive region (254) secured to the outer surface of the first portion of the impermeable barrier, the second adhesive region extending at least partially around the opening such that the second adhesive region is positioned at least partially between the opening and a second side portion of the fluid impermeable barrier opposite to the first side portion; and
adhering the second adhesive region to at least one of pelvic, suprapubic, or abdominal skin of the user on a second side of the penis.
